# EUROPEAN PATENT APPLICATION

(11) **EP 4 618 093 A1**
(43) Date of publication of application: **17.09.2025**
(21) Application number: 24162603.5
(22) Date of filing: 11.03.2024
(51) Int. Cl.: G16H 20/40, G16H 40/63

(54) **METHOD FOR OPERATING A DIALYSIS DEVICE TRAINING SYSTEM, DIALYSIS DEVICE TRAINING SYSTEM, MEDICAL SYSTEM AND AUGMENTED REALITY DEVICE**

(71) Applicant: Fresenius Medical Care Deutschland GmbH, 61352 Bad Homburg (DE)
(72) Inventor: Kipp, Sabine, 61352 Bad Homburg (DE); Heinze, Marcel, 61352 Bad Homburg (DE); Jakobi, Tim, 61352 Bad Homburg (DE); Witzel, Stephan, 61352 Bad Homburg (DE)
(74) Representative: Schwarz, Claudia

(57) **Abstract**

The present invention relates to a training of the user for a medical device, in particular a dialysis device. For this purpose, instructions for carrying out a task to be trained are computed and the user is provided with assistance for carrying out the task by means of an AR device. In particular, the AR device may further monitor the activities of the user so that a response of the medical device according to the activities of the user can be calculated and related virtual feedback is provided to the user.

## Description

The present invention relates to a method for operating a dialysis device training system, in particular a dialysis device training system with an augmented reality, AR, device. The present invention further relates to a dialysis device training system. Further, the invention relates to a medical system and an AR device. In particular, the present invention relates to computing indications for training employing an AR device.

Even though the present invention is described in the following by way of example with reference to a dialysis device, the general concept may be also applicable to other devices, in particular other medical devices, especially blood treatment devices.

More and more medical devices are becoming increasingly complex. An example of such a complex medical device is a dialysis device. Such medical devices require complex operations and configurations which have to be performed by the user. Further, numerous accessories and consumables should to be taken into account when operating medical devices.

Since improper operations could be dangerous for a patient, it is of great importance that a user, e.g. a nurse or doctor, is familiar with the device. In case of uncertainties, it is desirable that the user can be guided fast, safely and reliably through the tasks.

At present, the user has to read the manual of the respective device in order to learn to operate it. Further, even if the user may be trained by one or more human trainers, for this training purpose, the user and the trainer must be present at the same location. This can require a great effort and high costs. Further to this, human trainers are not always sufficiently available.

In view of this, there is a need for a training system for a medical device such as a dialysis device, which makes it possible to train a user even without the presence of a human trainer.

### Summary

The present invention provides a method for operating a dialysis device training system, a dialysis device training system, a medical system and an Augmented Reality device with the features of the independent claims. Further advantageous embodiments are subject matter of the dependent claims.

According to a first aspect, a method for operating a dialysis device training system is provided. Preferably, the method may be computer-implemented. The method employs an augmented reality, AR, device. The AR device is used by a user when operating a dialysis device. The method comprises a step of selecting a task to be trained. The task to be trained is to be performed on the dialysis device. The method further comprises a step of computing user instructions for guiding the user through the task to be trained, in particular when the user is operating the dialysis device. Further, the method comprises a step of monitoring activities of the user, in particular the activities of the user when operating the dialysis device. The activities of the user may be monitored by at least one sensor component, in particular a sensor component of the AR device. The method may further comprise to determine or to algorithmically calculate or process an assistance with a sequence of indications. The method further comprises a step of providing (in particular the determined) assistance. The provided assistance may be a computed assistance for carrying out the task to be trained. The assistance is generated or computed based on the computed user instructions and the monitored activities. In particular the provided assistance may comprise an output of indications on an output component, especially an output component of the augmented AR device. Generally, the term "assisting a user" refers to providing assistance by technical means, in particular by control of the AR device.

The first aspect relates to the claimed method. Features, advantages or alternative embodiments which are described or claimed with respect to the method can be also transferred or assigned to the other claimed objects (e.g. the training system, the medical system, the augmented reality device) and vice versa. In other words, the device can be improved with features described or claimed in the context of the method and vice versa. In this case, the functional features of the method are embodied by structural units of the apparatus or device or system and vice versa, respectively. Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the claimed device or apparatus claim is adapted to carry out the claimed method.

According to a second aspect a dialysis device training system provided. The dialysis device training system comprises an augmented reality, AR, device and a control device. The control device may be configured to execute a method according to the first aspect. In particular, the control device is configured to select a task to be trained on a dialysis device. The control device is further configured to compute user instructions for guiding the user through the task to be trained, in particular when the user operates the dialysis device. Further, the control device is configured to monitored activities of the user. The activities may be monitored, for example, by at least one sensor component, in particular a sensor component of the AR device. Especially, the activities of the user are monitored when the user operates the dialysis device by executing the computed user instructions. The control device is further configured to provide assistance for carrying out the task to be trained. The assistance is computed or generated based on the computed user instructions and the monitored activities. In particular, the assistance may comprise an output of indications on an output component, especially an output component of the AR device.

According to a further aspect, a medical system is provided. The medical system comprises at least one a dialysis device and a dialysis device training system according to the second aspect.

According to still another aspect, an AR device is provided. The AR device may be used in the dialysis device training system according to the second aspect. The AR device may comprise an output device and/or a sensor component. The output device may be configured to provide indications based on the determined assistance when the user is to be trained on the dialysis device. The sensor component may be configured to capture data related with the dialysis device. Additionally or alternatively, the sensor component may be configured to capture data related with an activity of the user operating the dialysis device.

According to still another aspect, a computer program is provided. The computer program comprises computer instructions for executing the method as described above.

The present invention is based on the finding that operating a complex medical device such as a dialysis device is a great demand for the users. A user has to study the manuals and has to be extensively trained to use such a device. Further to this, training on a real medical device may be necessary in order to make the user familiar with the device. However, human trainers are not always available. Further, human trainers usually require additional effort in terms of time and money.

The present invention therefore takes into account this finding and aims to provide an approach for a technical solution for training user on a medical device such as a dialysis device. In particular, the present invention aims to provide a solution which makes it possible to provide improved training to a user without the need of an additional human trainer.

For this purpose, the present invention provides a system for training user on a complex medical device by technical means such as an AR device and a related control system. In this way, not only a human trainer can be omitted, but such a system can also be customised to the needs and characteristics of the user to be trained.

The method according to one aspect, mentioned above, may comprise using a large language model, LLM. In particular, the LLM may be used to provide assistance to the user, e.g., in the form of calculating the indications. The LLM may be trained for acquiring the abilities, mentioned before by learning statistical relationships from text documents or other information sources or files during a computationally intensive self-supervised and semi-supervised training process. The LLM may be built with a decoder-only transformer-based architecture. Alternatively or in addition other architectures may be used, such as recurrent neural network variants and Mamba (a state space model). In particular, the LLM may be used, to get an answer to a question provided in relation to the dialysis device. The answer may be provided on the output component of the AR device or on an output component of the dialysis device or of a user's (e.g., mobile) device. The user can ask the system by using the LLM, for example, which part of the machine is currently in front of him, where to place a disposable, where a certain part of the machine (e.g. the blood pump) is located, or ask for help with the next step. The system recognises the situation in the room with the help of AR sensors and/or the at least sensor component, evaluates the user's verbal interaction of the user accordingly using the LLM and provides corresponding information such as AR highlighting, text, arrows and/or prepared videos and/or graphics to answer the verbal user enquiry. This can be done in any language if the LLM has the appropriate language capabilities. The system may respond automatically in the language of the enquiry.

An AR device in the context of the present invention may be understood any as kind of device providing AR features. For example, such an AR device may be an AR or mixed reality, XR, headset such as, e.g., Microsoft HoloLense. However, any other kind of AR or XR device may be possible, too. The AR device may be also any other kind of device providing AR features. For example, a smart phone, tablet computer or any other kind of device with a camera and a display may be used as an AR device. In general, any kind of device which has capabilities for providing an overlay of a real scene with additional (artificial) elements may be used as AR device. "Augmented" in the present context refers to the fact, that the real or physical scene may be augmented or enriched with additional information, which may be provided as virtual indications.

An output component in the context of the present invention may be any kind of component for outputting optical, haptic and/or acoustic indications. The output component may be implemented, at least in part, by the above-mentioned AR device. In particular, the output component may be part of the AR device. For example, the output component may comprise a display for providing optical information or indications. In particular, the output component may comprise a display device for overlaying a real scene with additional virtual elements/indications. For this purpose, a semi-transparent display may be used for providing additional indications on a really scene to which the display is pointed. Alternatively, a scene may be captured by a camera and/or a sensor component, and the image data from the camera and/or sensor component may be overlaid with additional virtual indications on the display. Alternatively or in addition the output component may be implemented on another device, in particular a mobile device and/or the medical device although it is referred to as "output component of the AR device". In the latter case the output component may be outsourced or transferred from the AR device to an AR-external device.

Generally, an indication may be any kind of data, provided or represented on the output component. An indication may be provided in different forms, in particular in an optical, acoustic, haptic and/or other form. The output component is selected accordingly (optical output, loudspeaker output, vibrational output etc.). Different output components may be combined.

Optical indications may be any kind of optical and/or graphical representation which can be used in the context of operating the medical device or related parts thereof (consumables, filters etc.). For example, an optical indication may be a graphical element, an icon, an image (e.g. a photo or a drawing of a component of the medical device), a line, arrow, box, circle or any other geometric element, a text box with text and/or other information, a video sequence, and avatar, etc. The indication, in particular the optical indication, may be a static indication and/or a dynamic indication such as a video sequence, a moving or turning graphical element, etc.

Acoustic indications may be, for example, a sound such as a beep or a warning tone, a sound sequence, melody or spoken text. The spoken text may be an artificially generated text message or a text messages recorded from the human being. However, any other kind of acoustic indication may be possible, too.

A haptic indication may be, for example, a vibration, shaking and/or any other haptic output.

A sensor component in the context of the present invention may be any kind of input component, in particular any kind of component for sensing an environmental property and/or for receiving user input, e.g., like a user interface, UI, graphical user interface, GUI etc. For example, the sensor component may comprise optical sensor elements such as at least one camera, at least one laser scanner or the like. The sensor component may alternatively or in addition comprise an acoustic sensor element such as a microphone, which can be used, for example, for recording a voice command of the user. The sensor component may alternatively or in addition comprise sensor elements such as a gyroscope, an acceleration sensor (for one or more dimensions) and/or a distance sensor or any other kind of sensor for sensing a position, pose or movement of the user. The sensor element may alternatively or in addition comprise one or more communication modules. For example, such a communication module may be a WIFI-interface, a Bluetooth-interface, an RFI D-reader or any other wireless or wired communication module. However, it is understood, that any other kind of the sensor element may be also comprised the by the sensor component.

The sensor component or parts thereof may be part of the augmented reality device. Alternatively or in addition the sensor component may be provided as external device, in particular external to the augmented reality device. The sensor component may be comprised in the medical device and/or in another device, at least in part. Date exchange is provided.The sensor component may be a part of a mobile device (e.g. smartphone, tablet etc.).

One or more optical sensor elements of the sensor component may be used for capturing images in the environment of the user. In particular, an optical sensor may capture image data of the environment to which the user is currently looking. The image data captured by an optical sensor may be used, for example, for identifying a medical device which is currently operated by the user. Especially, the image data may be used for identifying attachments and/or accessories which are mounted on the medical device or which are available in the environment of the medical device. For example, the medical device, the attachments or accessories may be identified based on a pattern recognition approach for identifying characteristic elements of the respective elements. It may be also possible to scan a code, e.g. a QR code or the like in order to identify an element. However, any other approach for identifying the device or a further component may be also possible.

The data from the sensor component, in particular from one or more - in particular optical - sensor(s) may be also used, for example, for identifying an operational setting of the medical device. The operational setting may refer to a current technical configuration and/or state of the medical device and/or properties of the medical device (like e.g. position of device, device parts and/or connected disposables and/or accessories, to be used for device operation). For example, image data may be analysed in order to identify a current setting of a control element and/or to analyse an output of the display on the medical device.

Further, data from the sensor component, e.g., from one or more (e.g., optical) sensor(s) may be used for monitoring and/or evaluating an operation of the user. For example, an optical sensor may capture image data of a hand of the user. Such image data may be used in order to analyse a current operation, for example a movement towards a control element, an operation for attaching or removing accessories, an action in connection with a patient, or any other manual operation of the user.

Image data from one or more optical sensors may be also used, for example, in order to evaluate an available space surrounding the user and the medical device. For example, dimensions of the room in which the user and the medical device are located may be determined. Accordingly, it may be possible to consider spatial constraints in case of limited space due to small rooms or further devices located in the environment.

Further, it may be also possible, to determine, based on image data and/or other available data, a spatial location of the user and/or the medical device as an operational setting. For example, it may be possible to determine whether the user and the medical device are located at a hospital, a medical facility, at the patient's home, etc. Furthermore, it may be also possible to determine additional infrastructure which would be available and/or which would be needed for operating the medical device as operational setting.

Interfaces or communication modules may be used for any kind of communication purpose. The communication may be performed by a wireless or wired communication. For example, a communication may be established with the medical device. Such a communication with the medical device may be used, for example, in order to determine a type of the medical device, determine a current configuration of the medical device, determine a status of the medical device, and/or obtain an error message from the medical device, etc. Further, communications may be established to one or more databases. For example, is possible to refer to a database in order to obtain additional information of a medical device, obtain information of a user, e.g. preferences of the user, a training status, etc., obtain patient information, obtain operational information in connection with the medical device, receive updates, e.g. from a manufacturer, receive supplementary information for assisting the user, etc. However, it is understood, that any other kind of information may be also obtained from one or more databases.

Interfaces or communication modules may be also used to establish any kind of communication with an artificial or human expert. In case of an artificial expert, information for assisting the user may be obtained from a local or remote computing device. In case of a human expert, a communication channel may be established with such a human expert in order to obtain further assistance from the expert and/or to provide the expert with information of the medical device and/or the current user. In such a case, the human expert may be located either close to the medical device and the user. For example, the expert and the user may be located in the same room. Alternatively, the expert may be located spatially separated in another room, another building, another city, country or continent. In case that the human expert is located spatially separated, the remote expert may be provided with any kind of appropriate information such as configuration of the medical device, status/failure messages of the medical device, attached or available accessories, preferences of the user, etc. The human expert may be also provided with further real-time information such as images captured by a camera, a voice communication with the user, etc. The human expert may be provided with such information, for example by any kind of output devices such as a loudspeaker, a headset, a smartphone or tablet computer, a monitor, a VR, AR or XR device, etc.

Alternatively or in addition, the human expert may use a second AR device where the same or a similar indications are provided compared to the output of the AR device, the user is using who has requested assistance or to whom assistance is to be provided. With this, the output on the AR device, the user is using and the second AR device the human expert is using are comparable in order to be able to simulate the situation of the user at the medical device as realistically as possible.

A task to be trained in the context of the present invention may be any kind of operation or action in connection with the related medical device or related parts thereof, like consumables. A task may require at least one activity and in particular a set or sequence of activities to be carried out manually by the user or automatically via instruction. Such a task may be, for example, preparing the medical device for a desired task/job (e.g. setup of the medical device, in particular dialysis machine, in particular prior to commissioning of the medical device). For this purpose, a user may have to mount one or more attachments or accessories on the medical device, insert or connect one or more consumables, make specific settings on the medical device or perform any other required operation. If necessary, particular data of a patient have to be taken into account. A task may be, for example, monitoring the medical devices during operation. For this purpose, the user may have to monitor the status messages of the medical device, monitor a filling level of the consumables, monitor flow rates, etc. In case that one of the parameters is out of tolerances, the user has to react on this in order to ensure safe operation. In another example, a task may be, for example, reacting in case of a failure or error message. For example, if the medical device detects a failure or problem, the user has to take the necessary steps (activities) in order to remove the reason for such a problem. However, it is understood, that a task may comprise any other kind of operation in connection with the related medical device, such as maintenance, normal operation, testing, or training.

A task to be trained may be specified or selected, for example, by the user, for instance by user input. The user input may be received on a user interface. The user interface may be deployed on the dialysis device and/or on the AR device and/or on a control device. Additionally or alternatively, a task may be determined fully or at least partly automatically. Further to this, it may be also possible that another person, for example a trainer or instructor, may select a task which should be executed by the user. It may be also possible to determine or select such a task to be trained at least partially in an automated manner. For example, it may be possible to refer to historic data, for example data stored in a related user database, in order to evaluate a current training level of the user and to identify an appropriate task which shall be trained. However, any other approach for an automated and/or computer assisted determination of a task to be trained may be possible, too.

Assistance may be any kind of assistance which could be used for assisting the user in operating the medical device. For example, such an assistance may comprise information for guiding the user through a desired or selected task, e.g. configuring the medical device, attaching accessories, adapting the medical device to a particular patient, operating in the medical device, performing the steps in response to a waring or error message, refilling of consumables, and/or performing maintenance operations, etc.

The method according to the present invention may comprise to calculate or determine algorithmically an assistance. The algorithmic determination of the assistance may define the content of the assistance in form of e.g., instructional messages or indications. The determined assistance is provided in accordance with the determined assistance scheme. The assistance is provided on the output component, which may be implemented at least partially on the AR device and optionally on related devices, such as mobile devices assigned to the user an/or the medical device and/or parts thereof. The assistance may comprise output of indications, For example, an assistance may comprise a description for a single step or a sequence of steps which should be performed by the user in order to execute the desired task. The assistance may comprise, for instance, information for an appropriate setting of the medical device, information for required additional components or consumables, or the like. The assistance may also comprise, for example, indications to one or more specific positions at the medical device or related components, indications of how to operate a control element of the medical device, specifications how to handle or grasp a particular component, or any other information related with the desired task to be executed. The assistance may comprise or refer to optical, acoustic or haptic elements. For example, the assistance may refer to or comprise haptic events such as a shaking or vibration in order to draw the user's attention. The assistance may refer to or comprise acoustic elements such as a beep, warning tone, melody, any other kind of sound or even a spoken text message. The assistance may further refer to or comprise optical elements such as arrows, lines, circles, boxes, icons, images, text messages, video sequences, etc., in order to guide a user through the related task. However, it is understood, that any other kind of assistance may be used in order to assist a user for carrying out a desired task.

Assistance can be provided to the user in any appropriate manner. In particular, the assistance may be provided to the user by an appropriate output component. The output component may comprise, for example, optic devices such as one or more displays for providing optical indications. The output component may also comprise acoustic elements such as a loudspeaker, a headphone or the like in order to provide acoustic information. Further to this, the output component may comprise any other kind of appropriate output element, example for providing haptic output.

The output component may be comprised, at least in part, by an AR device. For example, optical indications may be provided by means of a display of the AR device. Further, acoustic indications may be provided by means of an acoustic output element of the AR device. However, it may be also possible that the AR device is connected to one or more further output elements so that at least part of the desired indications could be also provided by such external output elements not comprised by the AR device itself, e.g., on the medical device and/or on a mobile device, which may be registered in the system. The output component may comprise a plurality of different output elements, each of which may be deployed on different devices, e.g., dialysis device and/or AR device.

The operations for selecting the task to be trained, computing the user instructions, monitoring the activities of the user and providing assistance may be performed, for example, by an appropriate control device. Such a control device may be realised, for example, by a processor and a memory communicatively coupled to the processor. The memory may be configured to store program code which can be read by the processor. The program code may cause, when executed by the processor, the control device to perform appropriate operations, in particular operation for executing at least part of the method for operating the dialysis device training system. The control device may comprise further components such as interfaces for receiving data and/or outputting a data. In particular, the control device may comprise interfaces for any kind of wireless or wired communication. Furthermore, the control device may comprise elements such as any kind of appropriate input and/or output devices, for example a user interface for interacting with the user. However, it is understood, that the control device may comprise also any other kind of appropriate components, if necessary.

Activities of the user may refer to any actions the user is executing with respect to the medical device, like e.g., handling, operating or moving the device or parts or accessories thereof, taking actions which cause an effect of the medical device, like clicking buttons on the device or on a user interface.

The control device may be a control device which is arranged separate to the medical device, in particular separate to the dialysis device. However, the control device may be also integrated, at least in part, within the medical device. Especially, the control device may be realised, for example, by appropriate components of the dialysis device. In this way, the control device may be fully or at least partially implemented by the component of the medical device.

In a possible embodiment, the selected task may be a mock task. A mock task may comprise mock operations, i.e. operations which are not executed in reality or not executed completely. A mock task comprise one or more operations for setting at least one operational parameter and/or entering at least one input on the dialysis device. In other words, to carry out such a mock task, the user does not really operate the medical device, but rather imitates activities which correspond to the desired operations. In this case, the method may comprise a step of detecting an operation of the user. The operation may be detected based on the monitored activities of the user. Further, the method may comprise a step of computing a virtual result state of the dialysis device in response to the detected activity of the user. In other words, the virtual result state of the dialysis device in response to the user activity is mimicked. The computed or virtual result state may be provided to the user by virtually overlaying the computed result state of the dialysis device. This may be performed, by an output component of the AR device. Accordingly, the virtual result of this user operation is provided to users without actually carrying out an operation by the dialysis device. Accordingly, an error by the user therefore does not lead to an actual malfunction of the dialysis device. Further, no consumables are used up and consumed, because the use of the consumables is only virtual or simulated.

In a possible embodiment, the selected task may be a task comprising one or more operations for mounting or demounting an accessory. Furthermore, the selected task may also comprise one or more operations for filling a medium in the dialysis device. In this case, the method may comprise a step of computing a virtual representation of the medium which is filled in and/or a virtual representation of the accessory which is mounted or demounted. The computed virtual representation may be provided to the user by an output component of the AR device.

Accordingly, operations with disposable mediums or products can be trained by a real medical device without wasting such disposable products. Furthermore, operations with sensible accessories can be trained without a risk of damaging such sensible accessories.

In a possible embodiment, the method may comprise a step of outputting a notification if a monitored activity of the user deviates from a prestored target activity for the task to be trained.. The notification may be output by at least one output component, in particular an output component of the AR device. In this way, the user is immediately notified in case of an erroneous operation. Accordingly, the user can recognise his failure and correct his operation in good time. This can prevent the user from getting into the habit of doing a wrong operation.

In a possible embodiment, the method may comprise a step of calculating a training progress score. In particular, the training progress score may be computed based on a calculated error rate. Such an error rate can be calculated by comparing the monitored activities of the user with prestored target activities. Additionally or alternatively, the training progress score may be computed based on a measured time for carrying out the task to be trained and/or a level of difficulty assigned to the task to be trained. In particular the training progress score may relate to a progression of a training level. In other words, the training progress score can be used to characterise or represent how good the current training affects the user. Hence, such a training progress score can be used for (dynamically) adapting the training provided to the user.

In a possible embodiment, the user instructions are computed based on the calculated training progress score. In particular, the user instructions may be newly computed if a training progression score deviates from a predefined threshold or range. For example, if it can be concluded from the computed training progress score that the current way of training the user does not further increase the training progress score, a scheme or algorithm for computing the user instructions may be modified. This makes it possible to adapt the training to the user in a manner so that a training effect can be enhanced. Accordingly, a better training effect can be achieved within a shorter period of time so that the efficiency of the training is increased.

In a further embodiment, the method may comprise a step of determining a viewing direction of the user. The viewing direction of the user may be determined based on sensor data, in particular sensor data detected by means of a sensor component, especially a sensor component of the AR device. For example, a camera may be used in order to monitor for the pupils of the user. In this case, the provided assistance may be adapted to direct the viewing direction of the user to a target viewing direction and/or a target position. For example, optical indications such as arrows, lines, items or the like may be provided in order to guide the user to a target viewing position. However, it may be also possible to provide haptic or acoustic indications, for example a spoken text in order to draw the user attention to a specific position on the medical device. In this way, it can be ensured that the user is focused on the relevant part of the medical device.

In a possible embodiment, the method may comprise a step for configuring a velocity for carrying out the task to be trained. For example, a user may manually specify the velocity for carrying out a task. However, it may be also possible to configure the velocity for carrying out a task in an automated manner, at least in part. For example, the availability for carrying out the task may be adapted depending on an (automated) determined skill level or training progress score. Accordingly, the assistance is provided to the user base on the configured velocity. In this way, the velocity of the task to be trained may be adapted to the needs of the user so that the user can follow the provided assistance without being over challenged or under challenged.

In a possible embodiment, the method may comprise a step of detecting a predefined user input. In particular, it may be possible to detect a predefined user gesture. Such a predefined user gesture may be, for example, a specific gesture of a hand or one or more fingers. The gesture may be a static gesture or a dynamic gesture such as a specific movement of a hand or finger. In this case, the provided assistance to the user for carrying out the task to be trained may be adapted based on the detected user input. For example, the provided assistance may jump to a specific position in the computed instructions based on the detected user input. Such a specific position may be, a starting point of the instructions, so that the instructions may be repeated from the beginning. However, it may be also possible to jump to any other appropriate position of the instructions, for example to repeat, a specific part of the instructions. Further, it may be also possible to skip in the provided assistance one or more instructions based on the detected user input. In this way, assistance for an already well-known part of a procedure may be skipped. This makes it possible to focus only on relevant parts of a training procedure. Further, a level of detail for providing the assistance may be adapted based on the detected user input. In this way, the user can easily adapt the level of detail to his current needs. For example, the level of detail may relate to an amount of information provided to a user for carrying out the desired task. Accordingly, such an amount of information may be enhanced or reduced based on the user input, especially based on a user gesture. The level of detail may also relate, for example, to activating or deactivating optical, acoustic or haptic assistance. For example, the user may individually activate or deactivate the optic, acoustic or haptic elements of the assistance based on user input, especially a gesture. However, any other kind of level of detail may be also adapted based on the user input. Furthermore, a new task to be carried out may be selected based on the detected user input. In this way, the user can easily select a new task to be trained based on user input, especially based on a simple user gesture.

In a possible embodiment, the method may comprise a step of computing a virtual character or person. Such a virtual character or person may be used to provide at least part of the assistance for carrying out the task to be trained. The virtual character or person may be provided by an output component of the AR device. In this way, such a virtual character person may simulate a human trainer. The virtual character a person may relate to a simulation of a complete person which may be of artificially generated. However, it may be also possible to generate only part of such a person, for example, an arm or a hand. Furthermore, it may be also possible to generate any kind of artificial object as a virtual character for assisting or training the user.

In a possible embodiment, the method may further comprise receiving an input message, representing input from a human trainer. The trainer may be located spatially close to the user, for example next to the user, especially in a same room. However, it may be also possible that the trainer is located spatially away, for example in another room, or even in another building or another country. In this case, the trainer may be connected to the user by an appropriate communication link. For example, the trainer may be provided with information of a camera, in particular, of the AR device, acoustic signals, for example, from a microphone of the AR device and/or output of the dialysis device. In this way, the trainer may grasp the current situation in order to assist the user. Especially, the computation of the user instructions and/or the assistance may be adapted based on the input message. For this purpose, the trainer may provide additional information which may be used for generating the assistance to a user or for directly providing the information of the trainer to the user by means of messaging.

In a possible embodiment, the activities of the user are determined based on pattern recognition of image data captured by a sensor component, in particular a camera of the AR device and/or of the dialyses device and/or based on machine learning applied to data obtained from the at least one sensor component, in particular a sensor component of the AR device and/or of the dialyses device. By analysing the user activities, the current operations of the user may be evaluated in order to identify a correct or erroneous operations for carrying out the desired task. Accordingly, this information may be taken into account in order to dynamically adapted the assistance to the user, modify the computation of instructions for user in order to carry out the desired task for to perform any other kind of operations in connection with an (immediate) indication for assisting the user.

In the following detailed description of the figures, non-limiting examples of embodiments with their features and further advantages are discussed with reference to the drawing.

### Brief description of the Figures

Fig.1: shows a schematic illustration of a medical system according to an embodiment;
Fig. 2: is a schematic illustration of a user with an AR device according to an embodiment;
Fig. 3: depicts a schematic illustration of a user with another AR device according to an embodiment;
Fig. 4: shows a schematic illustration of a flow diagram underlying a method according to an embodiment; and
Fig. 5: is a schematic illustration of scenario for training the user on a medical device according to an embodiment; and
Fig. 6: is a schematic illustration of scenario for training the user on a medical device according to another embodiment.

### Detailed description of the Figures

In the following, the invention is described in more detail with reference to embodiments in connection with the figures.

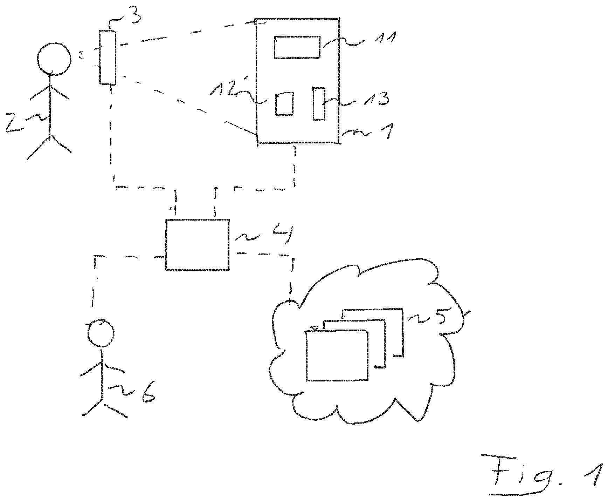

**Figure 1** shows a schematic illustration of a configuration with a medical system in connection with a training system for a medical device according to an embodiment. The medical system comprises a medical device 1, in particular a dialysis device or another blood treatment device. The medical device 1 may be operated by a user 2.

Blood treatment devices, in particular dialysis devices, are medical devices in which the blood of a patient is usually treated extracorporeally. For example, drugs may be added to the blood or blood components removed from it, blood may be heated or cooled. These processes are often monitored by sensors. Blood treatment devices have a variety of means for these purposes, such as pumps, valves, sensors, clamps or the like. Furthermore, medical disposables are often used for blood treatment. Such disposable articles are usually discarded after treatment for reasons of hygiene. Medical disposables, or so-called disposables, are, for example, tube sets for administering medical fluids such as blood or medicaments, filters, such as adsorber filters or dialysis filters, sachets for storing medicaments, and the like. These disposables are usually inserted by a user in the blood treatment device, or connected to the same and removed after treatment from the blood treatment device or separated therefrom.

In the example of figure 1, a medical device 1 is illustrated, wherein the medical device 1 comprises an input/output component 11, and at least one accessory component 12 representing any kind of attachment, accessory or the like, and a tank component 13 for receiving (liquid) consumables.

The high complexity of such medical devices 1 is a considerable challenge for the users of such a device. Thus, the user 2 must be properly trained before use. Further, it is desirable to monitor a user 2, when operating the device, in order to recognise a potential improper operation at an early stage. In addition, support to guide the user 2 during operation is also very beneficial.

In the medical system as shown in figure 1, an augmented reality (AR) device 3 may be used for assisting, monitoring and/or especially for training the user 2 in connection with an operation of the medical device 1. Any kind of appropriate device which could be used for AR purposes may be used. For example, the AR device may be any kind of AR headset or AR glasses. Furthermore, any other kind of device such as a tablet computer, a smart phone, etc. which can provide AR capabilities may be possible, too.

The AR device 3 may comprise or may be in in particular data connection with one or more output components. For example, an output component may provide optical/graphical output.

Such optic/graphical output may be provided, for example on a display. The display may be a non-transparent display on which an image of the environment, in particular an image of the medical device 1 or at least part of the medical device 1, is overlaid with further graphical elements. The display may also be a semi-transparent display so that the user 2 can look through this display in order to recognize the real environment. Furthermore, additional graphical elements may be provided by the semi-transparent display so that graphical indications on the semi-transparent display may provide assistance to the user 2 during operation of the medical device 1.

The AR device 3 may also comprise one or more further output components such as a loudspeaker, headphones or another device for outputting signals, in particular e.g. acoustic signals. In this way, acoustic signals such as a sound or melody or a spoken text message may be provided to the user 2. Further to this, any other kind of appropriate output component may be also comprised by or connected to the AR device 3. For example, the AR device 3 may also comprise or be connected to an output component for providing haptic signals such as a vibration, shaking or the like.

The AR device 3 may further comprise or be connected to one or more sensor components for sensing the environment and/or operations of the user 3. For example, the AR device we may comprise one or more image sensing elements such as a camera or the like for capturing image data. The AR device 3 may also comprise any other kind of sensor components, for example a gyroscope, acceleration sensors, velocity sensors, etc. for sensing a movement of the user 2. However, any other kind of appropriate sensing component may be possible, too. For example, a sensor component may also be a scanner, in particular a scanner for reading a QR code or barcode, an RFID reader, etc. Alternatively or in addition, the one or more sensor components may be provided separately form the AR device 3, e.g. as room sensors, or sensors of mobile devices or other sensor components, which are in data connection.

Preferably, are or at least some of the output components and sensor components can be implemented in the AR devices 3. However, as mentioned above, it may be also possible to use external output components and/or input components which are not implemented in the AR device 3.

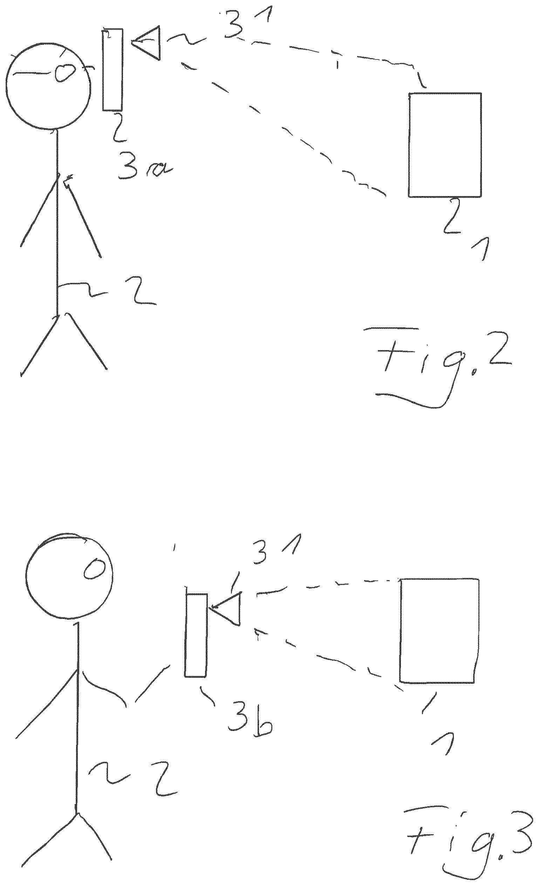

The other components, depicted in Fig., 1 are described below (after Fig. 3) in more detail.

**Figure 2** shows a schematic illustration of an AR device 3a, used when a user handles or operates the medical device 1, wherein the AR device 3a comprises a headset or AR glasses. In such an embodiment, the AR device 3a may be arranged on a head of the user 2. The user 2 may look through a semi-transparent display. Accordingly, the user 2 can still recognise the environment. In addition, graphical elements may be provided by the semi-transparent display so that the additional graphical elements may be shown at positions related to elements in the real world. In addition, the AR device 3a may also comprise further output elements such as loudspeakers or the like in order to provide acoustic output. Further, the AR device 3a may comprise a camera 31 in order to capture image data, in particular image data in a direction in which the user 2 is currently looking. Further, the AR devices 3a may comprise any other kind of sensor components for monitoring a movement of the AR device 3a and a corresponding movement of the user 2 or for acquiring any other kind of appropriate sensor data. The AR device 3a may also comprise any other kind of sensors, for example a microphone for receiving voice commands from a user. The sensor component, e.g. in the form of the camera 31 may alternatively or in addition be provided externally to the AR device 3, 3a.

**Figure 3** shows a schematic illustration of another type of AR device 3b. In this configuration, the AR device 3b may be realised by a handheld device such as a tablet computer or smartphone. The handheld AR device 3b may comprise a camera 31 for capturing image data, in particular during operation of the medical device 1. Further, the handheld AR device 3b may comprise a display for providing the captured image data in combination with additional graphical elements. In this way, the image data of the real environment may be overlaid with additional graphical elements. Alternatively, the handheld AR device 3b may comprise a semi-transparent display so that the user 2 can look through this semi-transparent display. In such a configuration, additional graphical elements may be provided on the semi-transparent display in order to provide additional information, in particular information related to one or more elements to which the user 2 is looking through the semi-transparent display. In any case, the handheld AR devices 3b may also comprise output components for providing acoustic and/or haptic output. Further, the handheld AR device 3b may comprise or be connected to any kind of sensor component, e.g. a microphone for sensing acoustic signals, in particular voice command of the user, or sensors for monitoring a movement of the handheld AR device 3b.

The AR device 3, 3a, 3b may be controlled, for example, by means of a control device 4. Especially, this control device 4 may be operated for training a user to on the medical device 1, as will be described in more detail below.

In figure 1, this control device 4 is illustrated as a separate component which is arranged external to the medical device 1. However, it may be also possible to implement this control device 4 into the medical device 1. Especially, it may be even possible that the functionalities of control device 4 may be realised by components of the medical device 1.

Control device 4 may receive sensor data from one or more sensor components, in particular sensor components of the AR device 3 or external sensor components, or another device related to AR device 3. Furthermore, control device 4 may also receive further information or a data from the medical device 1. For example, control device 4 may receive data relating to the configuration of the medical device 1, a current setting of the medical device 1, data for specifying a fill level of consumable of the medical device 1, operational data such as, for example, a flow rate, a pressure value or the like, status or error messages or any other information which are related to medical device 1.

Control device 4 may also establish a communication link with one or more external storage devices, in particular one or more databases 5. For example, such a database 5 may provide any kind of information related to the medical device 1. Such information may comprise, for example, any kind of data for specifying the medical device 1. For instance, control device 4 may receive a serial number of the medical device 1 and refer to a database 5 in order to obtain further information in connection with this serial number. It may be also possible to use image data, in particular image data captured by a sensor component of the AR device 3 in order to identify the medical device 1 or an attachment/accessory of the medical device 1. For example, a QR code or barcode may be scanned and control device 4 may refer to database 5 in order to identify the medical device 1 based on this code. Alternatively, it may be also possible to identify any appropriate features in an image representing the medical device 1, and to refer to a database 5 in order to identify the medical device 1 based on these features. However, any other approach for identifying a medical device 1, an accessory or attachment, or to retrieve any other information in connection with the medical device 1 may be possible, too.

Database 5 may also provide, for example, further relevant information such as recommended settings, a fill level of consumables, predefined operations for a user for operating the medical device 1 and/or any other kind of appropriate information in connection with medical device 1.

Database 5 may also provide any kind of information for specifying operations in order to carry out a specific task in connection with the medical device 1. Such a specific task may be, for example, a task for configuring the medical device 1, a task for operating or monitoring the medical device 1 during operation, a task in order to resolve a warning or failure of the medical device 1, a task for maintenance of the medical device 1 or even a task for repairing the medical device 1. However, any other kind of task may be specified, too.

Database 5 may also comprise further information in connection with one or more users 2. For example, the database 5 may store and provide any kind of preferences of a user 2. Such preferences may comprise, for example, a specific configuration, in particular a specific configuration for providing information by the AR devices 3, e.g. a colour scheme, a specification how detailed a support/assistance for the respective user 2 should be, a degree of knowledge or a skill level of the respective user 2, language skills, physical limitations of the user 2, or any other data in connection with the user 2.

Further, databases 5 may also provide any kind of information in connection with a patient which shall be treated by the medical device 1. Such information may comprise, for example, a physical characterisation of the patient, a desired therapy/treatment, medication of the patient or any other relevant data in connection with the patient.

In addition, database 5 may also provide any other kind of relevant information which could be useful in connection with an operation of the medical device 1.

Even though database 5 is illustrated in figure 1 as a single database 5, it is understood, that database 5 may be also realised by multiple separate databases. For example, separate databases may be provided for the properties of the medical device 1, data in connection with the operation of the medical device 1, the data in connection with a patient, the preferences of the user, etc. The individual databases 5 may be also located at different spatial locations. Further to this, it may be also possible to store at least some of the data in a local database within the control device 4.

As can be further seen in figure 1, it may be also possible to establish a connection to a further expert, in particular a further human expert 6. Such a further human expert 6 may be located close to the user 2 and/or the medical device 1. For example, the expert 6 and the user 1 may be located in a same room. However, it may be also possible that the expert 6 is located remotely. For example, expert 6 may be located in a separate room in a same building or any other position. For example, expert 6 may be located in another city, country or even continent.

Especially if the expert 6 is located somewhere outside the room with the user 2 and the medical device 1, expert 6 may be provided with any kind of appropriate information in connection with the medical device 1. For example, expert 6 may receive information of the current status, operational properties, warnings failures etc. of the medical device 1. Furthermore, expert 6 may also receive information in connection with a desired task to be executed currently in connection with the medical device 1. Expert 6 may also receive information in connection with the user 2 and/or the patient to be treated. However, any other kind of information may be also provided to the expert 6. For example, expert 6 may receive image data captured by one or more cameras, in particular one or more cameras of AR device 3. The information may be provided to the expert 6 in any appropriate manner, for example by means of a display, such as a monitor or a virtual reality (VR) or AR device.

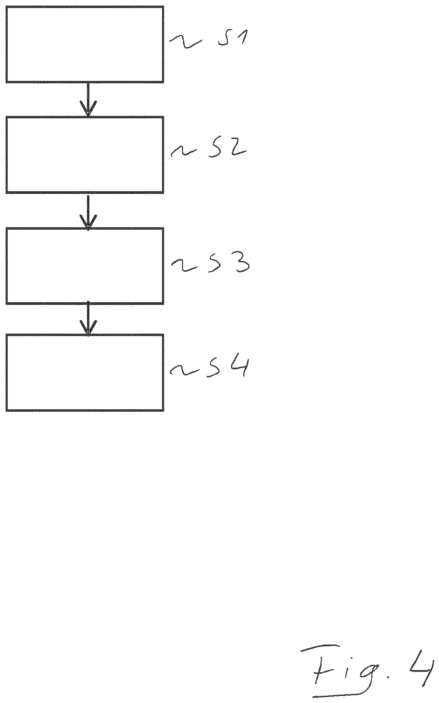

**Figure 4** shows a flow diagram for a method for operating a dialysis device training system, in particular a training system employing an AR device 3 associated with a medical device 1 according to an embodiment.

The term "on or at" refers to the fact that a training method may applied, when or during working with the medical device 1. All tasks or functions and/or actions to be performed in this respect may be supported. It is not necessarily required that the user 2 directly operated on the medical device 1 but may e.g. work at accessory devices, like e.g., disposables or related devices in the context of the (intended) treatment with the medical device. Further, assistance is also available in a setup or configuration phase of the medical device before the actual operation of the medical device.

Generally, the method may be implemented, for example, in the above-described medical system. Thus, the embodiments relating to systems, devices or apparatuses may comprise any kind of device, component or unit for implementing the features of the embodiments relating to a method. Accordingly, embodiments relating to the method may comprise any appropriate method steps for realising a feature as described in connection with an embodiment relating to a system, apparatus or device.

The method for operating such a dialysis device training system may be used, for example, for training a user 2 on the medical device 1. The training may be subjected to a general operation of the dialysis device 1, a specific task such as preparing, configuring or maintaining the dialysis device 1, or only a single step such as attaching an assembly or filling in a medium. Thus, in the following the expression "task to be trained" may relate to all these kinds of operations. Hence, a task to be trained may be any kind of operation in connection with the medical device 1 which shall be learned by the user 2. In particular, such a task to be trained may be an operation which usually is not related with a real treatment of a patient.

In a first step S1, a task to be trained by a user 2 on the dialysis device 1 is selected. Such a task to be trained may be selected manually by the user 2. For example, a user to may enter a desired task on a user interface or select such a task to be trained out of a number of available tasks to be trained. However, any other scheme for selecting a task to be trained may be possible, too.

Subsequently, in a step S2 instructions for guiding the user 2 through the task to be trained when operating the dialysis device 1 are computed. For example, it may be possible to refer to an appropriate database which stores the instructions in connection with a related task. Such a database may be a local database or a remote database, for example a storage device in a cloud. However, any other approach for determining the instructions may be possible, too. For example, the individual instructions may be computed based on a specific algorithm or by using a neural network.

The computed instructions may be instructions for operating a control element of the medical device 1. For example, an instruction may specify activating or deactivating a switch, turning a rotary knob, entering a command of value on a control panel or the like. An instruction may also relate to filling in a medium, especially a consumable into a tank of the medical device 1. Further, instructions may one or more operations for attaching or detaching accessories on the medical device 1. Instructions may also relate to operations for monitoring an output of the dialysis device 1, monitoring or controlling fill levels of consumables, a response to a specific message of warning on the dialysis device 1, or any other kind of activity in connection with the operation of the dialysis device 1.

In step S3, activities of the user 2 may be monitored. The activities may be monitored, for example, by at least one sensor component, in particular a sensor component of the AR device 3. For example, a camera of the AR device 3 may be used in order to capture images of the user 2 or at least one or more body parts of the user 2. For example, a camera may monitor the movement of the hand of the user 2. In this way, it may be possible to identify operation is performed by the user 2, in particular operations on the medical device 1 or in connection with the medical device 1. For instance, it may be possible to detect operation on a control panel, a switch or knob of the medical device 1. Furthermore, it may be also possible to detect operations in connection with connectivity for filling in a medium in a tank of the dialysis device 1, or an operation in connection with an attachment or detachment of an accessory.

It may be also possible to monitor an activity of the user in order to identify any kind of user input. For example, a hand or one or more fingers of the user may be monitored in order to identify predetermined patterns, for example a gesture. A gesture may be a static posture of a hand or a specific configuration of one or more fingers. A gesture may be also a dynamic motion of a hand or finger. In this way, specific commands may be associated with such a gesture of the user 2.

Further, it may be also possible to monitored the viewing direction of the user 2. For example, the pupils of the user 2 may be monitored by an appropriate sensor device, for example a camera. In particular, a sensor device of the AR device 3 may be used for this purpose. Accordingly, it may be possible to identify a direction or a position the user is currently looking at. This way, it may be possible to derive an element or a feature of the medical device 1 with which the user 2 is currently engaged. Especially, it may be possible to compare this viewing position or direction with a position or direction which is considered to be relevant in connection with the task to be trained, especially according to the computed instructions for carrying out tasks to be trained.

Further, in a step S4 assistance may be provided to the user 2. This assistance may be assistance for carrying out the task to be trained. In particular, the assistance may be generated based on the computed user instructions and the monitored activities. The providing assistance may comprise an output of indications on an output component, in particular of the AR device 3.

As already mentioned above, this assistance may be any kind of appropriate assistance which may be used for guiding the user 2 in order to perform the desired task to be executed. For example, the assistance may comprise optical indications such as lines, arrows, icons, images, text or the like. Furthermore, the assistance may comprise acoustic indications such as sound signals, melodies, spoken text or the like. The assistance may also comprise haptic indications, for example a shaking, vibration or the like.

In a possible embodiment, the assistance may be provided, at least in part, by means of a virtual assistant such as a virtual character or person. For example, an artificial character or person may be computed which is used to provide the user 2 with appropriate information. In possible implementation, an artificial person may be computed and/or computer animated. Such an artificial person may simulate a human trainer. Alternatively, any other kind of artificial character such as a static or animated object may be possible, too. Especially, it may be possible to establish a dialogue between the user 2 and the virtual assistant.

When the user 2 is trained on the medical device 1, the user 2 may have to perform multiple operations in connection with the medical device 1. It may be possible that the user 2 makes the input on the medical device 1 for real. In this case, the user may also recognise the real output of the medical device 1 in response to this operations. However, it may be also possible that the user input and/or the response of the medical device 1 may be simulated by the training system, in particular in connection with the AR device 3.

For example, it may be possible to identify user operations on the medical device 1 based on the monitored activities of the user. In such a scenario, the user does not operate the control elements of the medical device in real, but only simulates such an operation. The related operations are identified, and a reaction of the medical device 1 in response to this operation may be computed. Accordingly, it may be possible to compute a representation which simulates the reaction of the medical device 1 in response to the operation of the user. Such a simulated representation may be provided to the user, for example, by means of the AR device 3. For instance, the AR device 3 may provide an output to the user 2 which is overlaid to the respective positions on the medical device 1 so that the user may get an impression of a realistic reaction of the medical device 1 in response to his operations. Such an output may relate, for example, to a message on a control panel, a control indicator that lights up in a specific colour, a switch that is represented in a specific position, a sound signal, for example a control tone or warning message, or any other kind of the action of the medical device 1 in response to user operation. In this way, an operation of the medical device 1 may be simulated even without actively operating the medical device 1. Accordingly, even critical operations may be trained, for example operations which could lead to a damage of the medical device 1 in the event of an incorrect operation.

In a possible embodiment, it may be also possible to virtually train operations in connection with consumables and/or accessories. For example, it may be possible to generate a virtual representation of a container with a medium which shall be filled into the medical device. In such an example, the operation of filling the medium into the medical device 1, for example through an opening of the medical device 1 into a tank of the medical device 1, can be simulated. The one hand side, a virtual representation of the container with the medium may be provided to the user 2 and this virtual representation may move in line with the monitored activities of the user 2, in particular the movement of a hand which performs the operations for grasping the container and filling the medium into the medical device 1. Additionally or alternatively, it may be also possible to provide a virtual representation of the reaction of the medical device 1 in response to such a simulated operation for filling a medium into the medical device 1. For example, a virtual representation of an indicator for the fill level on the medical device 1 may be computed and provided to the user. Additionally or alternatively, any other reaction of the medical device 1 may be simulated, for example, a warning message may be output in case that a simulated operation would result in an overfilling of the medical device 1. Further to this, it may be possible to compute the consumption of such a medium during the operation of the medical device 1 and to calculate the point in time when the amount of the consumable runs out. This connection, the amount of consumable which is filled into the medical device 1 may be calculated based on the monitored activities during the operation for filling in this consumable into the medical device 1.

Further to this, it may be also possible to train the attachment or detachment of accessories based on virtual representations. For example, the user may be provided with a virtual representation of an accessory by means of an output component, in particular an output component of the AR devices 3. Accordingly, the user may perform activities which are intended to attach or detach such an accessory. In this connection, the behaviour of the accessory in response to the monitored activities of the user 2 may be calculated, and the result of the activities of the user 2 may be simulated. Accordingly, the user may be provided with an output which represents the result of his operations in form of a virtual result state of the medical device. For example, a virtual accessory may be provided to the user at an appropriate position of the medical device 1 after successfully mounting the respective accessory. Alternatively, in case of a failure, a related damage of the accessory may be computed and provided to the user. In this way, the user can be trained on the operations for mounting or demounting such accessories even without the risk of damage in case of a failure.

When using the medical device 1, the user can also be supported by a large language model (LLM), as known from ChatGPT, for example. The user may interact with such an LLM verbally. For example, a spoken request such as a phrase or question may be received and provided to the LLM. For this purpose, the request may be received by an input device, in particular an input device of the AR device 3, such as a microphone or the like, converted into text by a speech-to-text module and provided to the LLM. However, any other kind of input, such as entering the text by an input terminal, e.g. by means of a keyboard or the like, may be possible, too. The LLM may provide an appropriate response to the input of the user. This response may be provided to the user by an acoustically output, for example a synthetically generated voice output, by providing a text message, providing any other kinds of indication such as lines, arrows, symbols, icons, images and/or video clips. For this purpose, an appropriate output component of the AR device 3 may be used. The output may relate to output which is dynamically generated by the LLM in response to the request of the user. However, the output may also comprise, for example prestored elements which have been generated in advance. For example, the output may comprise prestored images such as images of the medical device 1 in a specific configuration, images of a specific part of the medical device 1, a video clip for illustrating a specific operation of any other kind of data which has been prepared in advance.

When generating the output, the LLM may take into account the language of the user. Accordingly, the LLM may provide the response in the same language as used by the user for formulating the request. For example, when the user asks a question in English, the LLM may also provide the response in English, and when the user asks a question in German, the LLM may provide the response in German. However, it may be also possible to limit the processing and/or the generation of the response to one or more specific languages. Furthermore, it may be also possible to provide a response of the LLM to a specific language regardless of the language in which the input was formulated. For example, the LLM may always provide a response in a language corresponding to which the medical device 1 is set.

The dialogue with the LLM may be used for any kind of interaction, in particular for any kind of assistance which can help the user in operating the medical device 1. For example, the user may ask which component or which part of the medical device 1 is currently located in front of him. For this purpose, it may be also possible to take into account supplementary information.

For example, an input component, in particular input component of the AR devices 3, may detect a viewing direction of the user and/or the environment of the user, in particular it may be possible to detect/identify elements or objects in the environment of the user. Accordingly, this information may be also taken into account when the LLM generates a response to the request of the user.

The user may also ask, for example, how to perform a desired operation, for example how to attach an accessory or disposable, or how to execute a next step of the desired operation or the user may ask where a specific element of the medical device is located. In order to generate a response to such a request, it may be possible to take into account any kind of additional information such as information acquired by a sensor component, in particular a sensor component of the AR devices 3. For example, image data of the environment may be captured by an optical sensor component such as a camera. The image data may be processed in order to identify the medical device 1, the configuration of the medical device 1, accessories of the medical device, a spatial relationship between the user and the medical device 1, a current operation of the user or to detect a spatial position of a part of the body of the user, such as the current position of a hand of the user. Further, it may be also possible to receive supplementary information from any other kind of data source. For example, it may be also possible to receive information about the current configuration of the medical device 1, a current state of the medical device 1, operation parameters of the medical device 1 or any other property of the medical device 1 via a wired or wireless communication link.

Accordingly, the user may be assisted by a verbal interaction with the LLM in order to carry out a desired task and/or to get more information about the medical device 1. The information provided by the LLM do not necessarily have to refer only to a task currently being executed by the user. It is also possible for the user to interact with the LLM to obtain any other kind of information, for example general information in connection with the medical device 1.

During the training, the activities of the user 2 may be monitored and analysed. Accordingly, it may be possible to evaluate whether or not the user 2 follows the instructions according to the provided assistance and whether the user 2 carries out the desired operations correctly. For example, it may be possible to compare the monitored activities of the user 2 with target operations, in particular pre-stored target operations. In case that the operation of the user 2 deviate from the target operations, an appropriate signalling may be provided to the user 2. Such a signalling may be, for example, an optical signalling, a haptic signalling or an acoustic signalling. Accordingly, the user can immediately recognise his failure and correct his operation.

Furthermore, the analysis of the operation of the user and the assessment based on a comparison with target operations may be used in order to evaluate a training progression of the user 2. For example, an error rate may be computed. Such an error rate may relate to a number of deviations between the operation of the user 2 and the related target operations. The error rate may be an absolute number of deviations between the activities of the user 2 and the target operations. Furthermore, it may be also possible to consider a relative value which takes into account additional properties, such as a complexity level, a period of time, a number of repetitions which have been already performed for a same task to be trained, or any other appropriate feature.

The analysis of the matching between the activities of the user 2 and the related target operations of may be not only used for calculating a simple error rate, but even for a more complex training process score. Such a training process score may consider a progression of a training level, e.g. how good or fast a user 2 is learning during the training process. Such a training process level may take into account the error rate, a period of time the user needed for carrying out the task to be trained, a (predetermined/prestored) complexity level of the task to be trained and/or any other appropriate parameter. Especially, it may be possible to adapt a procedure for training a task based on the training progression, in particular based on the calculated training process score. For example, a velocity for providing the assistance to the user 2 may be adapted based on the calculated training process score. In a possible example, an unexperienced user with a low training process for may be provided with assistance in a low velocity, and the velocity is increased with increasing training process score. Furthermore, it may be also possible to adapt a degree of detail for providing assistance based on the calculated training process score. For example, an unexperienced user may be provided with more detailed information and/or a large number of information, with an increasing training process score, the amount of information provided to the user for carrying out a desired task may be reduced. Furthermore, it may be also possible, for example, to skip at least some substeps in a task, if the calculated training process score exceeds a predetermined threshold. However, any other approach for adapting the assistance based on the training process score may be possible, too.

The adaption of the assistance or the computation of the instructions for carrying out the task to be trained may be triggered, for example, if the currently calculated training process score deviate from a specific range or exceeds/decreases a threshold value.

Further to this, the training of the user 2 may be also adapted manually based on the demands of the user 2, in particular demands which may be specified by the user 2. For example, the user may specify a velocity of the training process, in particular a velocity with which the assistance for carrying out the task to be trained shall be provided. The user may also specify further features such as a language in which the assistance is provided, optical and/or acoustic properties for providing the assistance or any other kind of references. The preferences may be stored and retrieved, for example, from a local or remote storage.

The preferences and any other kind of user input may be provided, by means of an appropriate user interface. Furthermore, gestures of the user may be used for interacting with the training system. In particular, it may be possible to interact with the training system based on the gesture of a hand or fingers as already described above. The gestures may be also used, for example, in order to control the provision of the assistance. For example, a specific gesture may be used for selecting a task to be trained. One or more further gestures may be used, for example, for configuring the provision of the assistance such as adapting the velocity or a degree of detail when providing the assistance. Further gestures may be used, for example, in order to "navigate" through the assistance for carrying out the task to be trained. For example, a gesture may be used in order to switch back to the beginning of the task to be trained or to a specific position within the task to be trained. A further gesture may be used, for example, for skipping a specific step or sub-task or for terminating the task to be trained. However, any other action may be also controlled based on gestures of the user 2.

Furthermore, it may be also possible that the automated training, may be monitored, controlled or supervised by a human trainer. The trainer may be a local trainer which is close to the user 2, for example in a same room. However, it may be also possible that the trainer may be located remote from the user 2. Tn this case, the trainer may be provided with relevant information via a communication link. For example, a voice communication between the user 2 and the trainer may be established, the trainer may be provided with image data from one or more camera, especially, of the AR device 3 or any kind of further relevant information. The trainer may further assist the user when carrying out the task to be trained, the trainer may specify a specific task to be trained, or may activate a specific property, for example a simulation of an error or a particular operational state of the medical device. However, any other kind of interaction between the trainer and the user 2 and/or the medical device 1 may be possible, too.

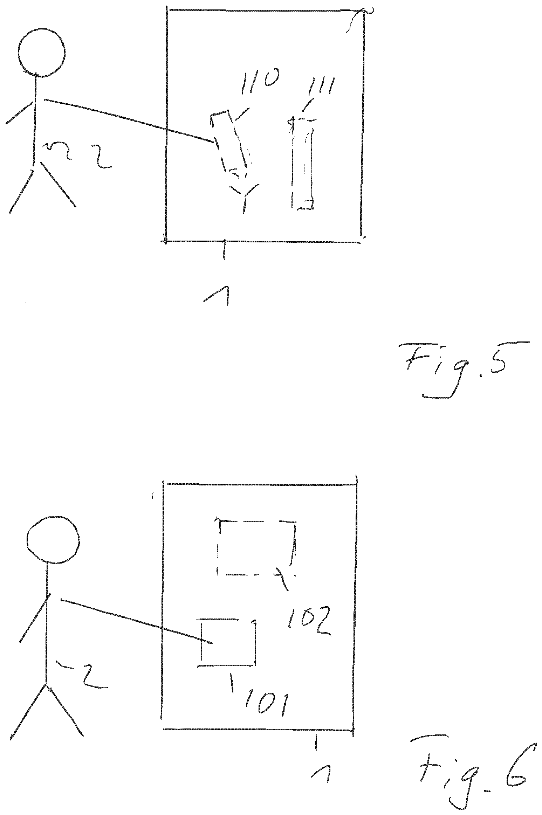

**Figure** 5 shows a schematic diagram illustrating an exemplary training situation according to an embodiment. In the example of figure 5, the user 2 is operating a control panel 101. Especially, the sensor component of AR devices 3 may monitored the activity of the user in order to determine the respective activities of the user to on the control panel 101. In response to this, a reaction of the medical device 1 is computed and respective output is provided to the user by a virtual overlay 102. In other words, the medical device 1 does not perform any operations in real, but the respective response is calculated and simulated to the user by virtual overlay 102.

**Figure 6** shows a schematic diagram illustrating an exemplary training situation according to a further embodiment. In the example of figure 6, the user 2 simulates a filling operation by means of the virtual representation 110 of a container comprising a medium. The virtual representation 110 may be provided by an output component of an AR device 3. Further, a sensor component, in particular a sensor component of AR device 3 may monitor the operation of the user 2. Accordingly, the filling operation may be simulated and a related fill level may be calculated. This calculated fill level may be also provided to the user by a virtual representation 111. Accordingly, the user may perform realistic operations in connection with the medical device 1 even though no real consumable is required.

Summarising, the present invention relates to a training of the user for a medical device, in particular a dialysis device. For this purpose, instructions for carrying out a task to be trained are computed and the user is provided with assistance for carrying out the task by means of an AR device. In particular, the AR device may further monitor the activities of the user so that a response of the medical device according to the activities of the user can be calculated and related virtual feedback is provided to the user.

## Claims

1. Method for operating a dialysis device training system, employing an augmented reality, AR, device (3) associated with a dialysis device (1), the method comprising the steps of:
- selecting (S1) a task to be trained on the dialysis device (1);
- computing (S2) user instructions for guiding a user (2) through the task to be trained when operating the dialysis device (1);
- monitoring (S3), by at least one sensor component, activities of the user when operating the dialysis device (1), in particular when executing the computed user instructions;
- providing (S4) assistance for carrying out the task to be trained based on the computed user instructions and the monitored activities, wherein providing (S4) assistance comprises an output of indications on an output component of the AR device (3).

2. Method according to claim 1, wherein the selected task is a mock task comprising mock operations, and the selected mock task comprises an operation for setting at least one operational parameter and/or entering at least one input on the dialysis device (1), wherein the method comprises
- detecting an operation of the user (2) based on the monitored activities of the user (2);
- computing a virtual result state of the dialysis device (1) in response to the detected activity of the user (2);
- virtually overlaying the computed virtual result state of the dialysis device (1) without actually carrying out an operation by the dialysis device (1).

3. Method according to claim 1 or 2, wherein the selected task is a task comprising operations for mounting or demounting an accessory and/or filling a medium, and wherein the method comprises:
- computing a virtual representation of the medium which is filled in and/or a virtual representation of the accessory which is mounted or demounted; and
- providing the computed virtual representation by an output component of the AR device (3).

4. Method according to any of preceding claims, comprising outputting a notification by at least one output component, in particular an output component of the AR device (3), if the monitored activities of the user (2) deviate from a prestored target activities for the task to be trained.

5. Method according to any of preceding claims, comprising calculating a training progress score, wherein the training progress score is computed based on a calculated error rate which is calculated by comparing the monitored activities of the user (2) with prestored target activities, a measured time for carrying out the task to be trained and/or a level of difficulty assigned to the task to be trained, in particular, wherein the training progress score represents a progression of a training level.

6. Method according to preceding claim 5, wherein the user instructions are computed based on the calculated training progress score, in particular wherein the user instructions are newly computed if a training progression score deviates from a predefined threshold or range.

7. Method according to any of preceding claims, comprising
- determining a viewing direction of the user (2) based on sensor data, detected by means of a sensor component,
wherein the provided assistance is adapted to direct the viewing direction of the user (2) to a target viewing direction, in particular a target position, in particular according to the selected task.

8. Method according to any of preceding claims, comprising a step for configuring a velocity for carrying out the task to be trained, wherein the assistance is provided according to the configured velocity.

9. Method according to any of preceding claims, comprising detecting a predefined user input, in particular a predefined user gesture,
wherein the provided assistance for carrying out the task to be trained is adapted based on the detected user input,
in particular, wherein the provided assistance jumps to a specific position in the computed user instructions based on the detected user input, the provided assistance skips one or more user instructions based on the detected user input, a level of detail for providing the assistance is adapted based on the detected user input, and/or a new task to be carried out is selected based on the detected user input.

10. Method according to any of preceding claims, comprising a step of computing a virtual character or person, wherein the virtual character or person is used to provide at least part of the assistance for carrying out the task to be trained, and wherein the virtual character or person is provided by an output component of the AR device (3).

11. Method according to any of preceding claims, comprising receiving an input message, wherein the input message represents input from a trainer, wherein computing (S2) the user instructions and/or providing (S4) the assistance is adapted based on the received input message.

12. Method according to any of preceding claims, wherein based on the monitored activities of the user, actions are determined based on pattern recognition and/or based on machine learning applied to data obtained from the at least one sensor component.

13. Dialysis device training system, comprising
an augmented reality, AR, device (3);
a control device (4), configured to execute a method according to any of preceding claims 1 to 12 and configured to:
- select a task to be trained on a dialysis device (3);
- compute user instructions for guiding the user (2) through the task to be trained when operating the dialysis device (1);
- monitor, by at least one sensor component, activities of the user (2) when operating the dialysis device (1), in particular when executing the computed user instructions;
- provide assistance for carrying out the task to be trained based on the computed user instructions and the monitored activities, wherein providing assistance comprises an output of indications on an output component of an AR device (3).

14. Medical system, comprising:
at least one dialysis device (1); and
a dialysis training system according to claim 13.

15. Augmented reality, AR, device (3) for use in a dialysis device training system according to claim 13, comprising:
an output device configured to provide indications based on the determined assistance when the user (2) is to be trained on the dialysis device (1), and/or
a sensor interface to a sensor component configured to capture data related with the dialysis device (1) and/or an activity of a user (2) operating the dialysis device (1).
